# EUROPÄISCHE PATENTSCHRIFT

(11) **EP 3 332 836 B1**
(45) Veröffentlichungstag und Bekanntmachung des Hinweises auf die Patenterteilung: **03.07.2019**
(21) Anmeldenummer: 16203193.4
(22) Anmeldetag: 09.12.2016
(51) Int. Cl.: A61N 1/375

(54) **DURCHFÜHRUNG EINES IMPLANTIERBAREN MEDIZINELEKTRONISCHEN GERÄTS**
FEEDTHROUGH OF AN IMPLANTABLE MEDICAL ELECTRONIC DEVICE
TRAVERSÉE D'UN APPAREIL MÉDICAL ÉLECTRONIQUE IMPLANTABLE

(43) Veröffentlichungstag der Anmeldung: 13.06.2018
(73) Patentinhaber: BIOTRONIK SE & Co. KG, 12359 Berlin (DE)
(72) Erfinder: Kronmüller, Daniel, 90409 Nürnberg (DE); Arnold, Michael, 91056 Erlangen (DE)
(74) Vertreter: Galander, Marcus

(56) Entgegenhaltungen:
- US-A1- 2007 239 222
- US-A1- 2008 119 906
- US-A1- 2009 079 519
- US-A1- 2014 262 493
- US-A1- 2014 272 457

## Beschreibung

Die Erfindung betrifft eine Durchführung eines implantierbaren medizinelektronischen Geräts.

Eine solche Durchführung ist insbesondere geeignet für elektromedizinische Implantate, wie implantierbare Herzschrittmacher, Defibrillatoren, Cardioverter, Nerven- und Hirnstimulatoren, Hörgeräte, implantierbare Medikamentenpumpen oder sonstige elektrische aktive Implantate, welche ein hermetisch dichtes Gehäuse umfassen, sowie Batterien mit hermetisch dichtem Gehäuse für diese elektronischen Implantate. Des Weiteren betrifft die Erfindung ein implantierbares medizinelektronisches Gerät mit einer solchen Durchführung.

Die meisten praktisch bedeutsamen implantierbaren elektromedizinischen Geräte (IMDs) sind dazu vorgesehen, über geeignet platzierte Elektroden elektrische Impulse an reizbares Körpergewebe abzugeben. Ferner können viele Geräte elektrische Impulse und Reize im Körper des Patienten gezielt messen und über einen längeren Zeitraum aufzeichnen oder auswerten, um eine individuell angepasste Therapie zu wählen und den Erfolg der Behandlung in vivo zu überprüfen.

Um diese Funktionen auszuführen, sind im Gehäuse des Gerätes elektronische/elektrische Funktionseinheiten zur Erzeugung und Messung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Die Aufgabe einer Durchführung besteht darin, die elektrischen Signale durch das hermetisch geschlossene Gehäuse zu führen und so einen elektrischen Kontakt der Elektronik im hermetisch dichten Gehäuse mit den Elektroden im Körper des Patienten zu ermöglichen. Hierfür bietet die Durchführung eine Schnittstelle auf der Körperseite sowie eine Schnittstelle auf der Elektronikseite zum Anschluss der elektrischen Signale. In vielen solchen Durchführungen werden hierfür Anschlussstifte eingesetzt, die im Geräteinneren mit der dort befindlichen Leiterplatte oder einem ähnlichen Leitungsträger kontaktiert werden und die Signale durch das Gehäuse führen, bei vielen IMDs speziell in ein Kopfteil (Header) enden.

Üblicherweise weisen die Durchführungen der elektromedizinischen Geräte (IMDs) einen Flansch auf, mit dem sie in einer Gehäusewand des elektromedizinischen Implantats eingesetzt sind, vorzugsweise durch ein thermisches Fügeverfahren wie Schweißen, Hartlöten (Brazing) oder Weichlöten (Soldering). Im Gehäuse befindet sich eine elektronische Einrichtung, normalerweise unter anderem mit einer Platine, welche in der Lage ist, elektrische Signale zu verarbeiten oder zu senden.

Die Durchführung weist typischerweise mindestens eine Durchführungshülse auf, einem die mindestens eine Durchführungshülse umgebenden Flansch, in der mindestens ein Anschlussstift sitzt, welcher von der Durchführungshülse umgeben ist. Der Anschlussstift erstreckt sich durch den Flansch und die Durchführungshülse hindurch von einem inneren Ende im Inneren des Gehäuses zu einem äußeren Ende, welches außerhalb des hermetisch dichten Gehäuses liegt.

In der Regel sind der Anschlussstift mit der Durchführungshülse und/oder die Durchführungshülse mit dem Flansch mittels einer Lotverbindung, vorzugsweise bei Verwendung von metallisch beschichteten Durchführungshülsen (beispielsweise eine Niob-Beschichtung) mittels eines Goldlotes oder bei Verwendung unbeschichteter Durchführungshülsen mittels eines biokompatiblen Glaslotes (Typ 8625 der Firma Schott), verbunden.

Im Hinblick darauf, dass das äußere Ende der Anschlusselektrode bei einem medizinischen Implantat mit dem das Implantat umgebenden Körpergewebe in Kontakt kommen kann, sind die Anschlussstifte in der Regel aus einem biokompatiblen Material, wie beispielsweise Niob (Nb), Platin (Pt), Iridium (Ir), Platin/Iridium-Legierungen (Pt/Ir), Tantal (Ta), Titan (Ti), Zirkonium (Zr), Hafnium (Hf), medizinischer Edelstahl (z. B. 316L) oder Legierungen aus diesen Materialien gefertigt. Möglich sind als Materialien für den Anschlussstift auch Fe-Ni, FeNiCo, FeCr, Molybdän (Mo), Wolfram (W), Chrom (Cr), FeCr, Vanadium (V), Aluminium (A1) oder anderen Legierungen aus diesen Materialien.

Die US 7,747,321 B2 offenbart, wie in Fig. 1 gezeigt, einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind am gehäuseseitigen Ende durch eine entsprechende Bohrung in der Leiterplatte gesteckt und mit dieser weich verlötet. Sie umfassen einen Draht-Kern, etwa aus Tantal, Niob, Titan, Molybdän oder Kupfer und eine oxidationsbestätige Ummantelung aus einem biokompatiblem Material, etwa Gold, Platin, Titan oder Ähnlichem.

Die Verbindung der Anschlussstifte mit dem im Gehäuse angeordneten Leitungsträger erfolgt typischerweise mittels Schweißen, Bonden, Crimpen, Weichlöten oder Klebens. Insbesondere das Weichlöten (Reflow-Löten) hat in den letzten Jahren stark an Bedeutung gewonnen, da es ein sehr wirtschaftliches Verfahren ist. Die für die Anschlussstifte üblicherweise verwendeten Materialien sind für dieses Verbindungsverfahren schlecht geeignet, so dass verschiedene Strategien entwickelt wurden, um die Weiterverarbeitung der Durchführung zum Anschluss an den Leitungsträger in einem hinreichend beherrschbaren und kostenmäßig vertretbaren Prozess zu gewährleisten. Entsprechende Ansätze sind unter anderem in der EP 2 371 418 A1 oder US 2016/0001387 A1 der Anmelderin oder auch der EP 2 529 790 A1 offenbart.

Die Verbindung der Anschlussstifte mit den Elektroden bzw. dem Stecker für die Elektroden im Header erfolgt typischerweise mittels Schweißen. Andere Fügeverfahren wie z. B. Weichlöten, Hartlöten, Boden, Kleben, Crimpen sind ebenfalls möglich, werden in der Praxis aber selten angewendet.

Im Zuge der Miniaturisierung und der Fortschritte der Elektronik hat sich die Weichlöttechnik (Solder) in den letzten Jahren rasant gewandelt. Manuelle Bestückungsprozesse wurden zunehmend von vollautomatischen Pick&Place-Automaten verdrängt und die Through Hole Technology (THT) wurde allmählich durch die Oberflächenmontage (SMT) ersetzt. Dies ermöglicht kleinere, kompaktere Schaltungen und führt somit schrittweise auch zu kleineren, patientenverträglicheren IMDs. Um die vielen Vorteile der SMT erreichen zu können, müssen Durchführungen die Anforderungen an ein oberflächenmontiertes Bauteil (SMD) genügen.

Insgesamt haben sich bei den Montageprozessen für Durchführungen der genannten Art, insbesondere bei Anwendung der SMT, gewisse Probleme, wie vor allem eine erhebliche Anfälligkeit der Durchführungen, speziell der Anschlussstifte, für Deformation und Beschädigungen, gezeigt. Um diesen vorzubeugen, werden spezielle Maßnahmen zum Schutz der Enden der Anschlussstifte ergriffen, und durch geeignete Prüfverfahren ist sicherzustellen, dass ein maßgenauer und zuverlässiger elektrischer Kontakt mit dem Leitungsträger hergestellt werden kann. Beide Arten von Maßnahmen machen die Weiterverarbeitung der bekannten Durchführungen aufwändig und relativ kostspielig.

Das Dokument US 2014/0272457 A1 offenbart ein Implantat mit einer Durchführung nach dem Oberbegriff des Anspruch 1.

Die Dokumente US 2008/0119936 A1 und US 2009/0079519 A1 beschreiben weitere Ausführungen für Durchführungen in medizinischen Implantaten.

Der Erfindung liegt die Aufgabe zugrunde, eine hermetisch dichte Durchführung für Implantate aus einem körperverträglichen Material zur Implantat-Außenseite hin anzugeben, die auf herstellungstechnisch einfache Weise an ihrem inneren Ende mit der dort angeordneten Elektronik kontaktverbindbar sind, insbesondere mittels des Reflow-Lötens.

Diese Aufgabe wird durch eine Durchführung mit den Merkmalen des Anspruchs 1 gelöst. Zweckmäßige Fortbildungen des Erfindungsgedankens sind Gegenstand der abhängigen Ansprüche.

Die Erfindung schließt den Gedanken ein, von der bisherigen Herangehensweise mit Schutz- und Prüfmaßnahmen für die aus den Durchführungen herausstehenden Anschlussstifte grundlegend abzugehen und stattdessen die potentielle Fehlerquelle von vornherein zu beseitigen. Demnach weist das oder jedes primäre Anschlusselement mindestens ein Ende auf, welches der Oberfläche des Isolierkörpers gegenüber in den Isolierkörper hinein versetzt ist. Hierdurch wird die Anfälligkeit der bekannten Durchführungen für Deformationen und Beschädigungen der Enden der Anschlussstifte völlig beseitigt, und aufwändige Schutzmaßnahmen und Prüfvorgänge können ersatzlos entfallen.

Gemäß einem Aspekt ist am Ende des oder jedes primären Anschlusselementes eine Aufweitung des entsprechenden Durchlasses als Materialreservoir vorgesehen. Das Materialreservoir ist, je nach konkreter Verbindungstechnologie zur Verbindung zwischen Anschlussstiften und Isolierkörper einerseits und Anschlussstiften und Leitungsträger andererseits, mit geeigneten Materialien oder Materialkombinationen bzw. -schichten mindestens teilweise gefüllt und erleichtert die präzise Materialzuführung für die jeweiligen Prozessschritte.

In zweckmäßigen Ausführungen ist die Aufweitung zylindrisch, kegelstumpf- oder schüsselförmig ausgebildet. Grundsätzlich sind auch andere Formen der Aufweitung möglich, die hier Erwähnten sind aber aus derzeitiger technologischer Sicht die Zweckmäßigsten.

In einer Ausführung ist die Aufweitung des Durchlasses teilweise mit einem Hartlot, welches zugleich das Anschlusselement im Durchlass umgibt, ausgefüllt, und auf der Oberfläche des Hartlotes und optional der gehäuseseitigen Endfläche des Anschlusselementes ist ein Weichlot oder Sintermaterial vorgesehen. In einer Ausgestaltung sind in der Aufweitung neben dem Hartlot ein erstes, nicht weichlötbares Sintermaterial und ein zweites, weichlötbares Sintermaterial vorgesehen. In einer weiteren Ausgestaltung sind in der Aufweitung ein erstes, höher schmelzendes Hartlot und auf dessen Oberfläche ein zweites, niedriger schmelzendes Hartlot vorgesehen.

Es ist das gehäuseseitige Ende des oder jedes Anschlusselementes gegenüber der gehäuseseitigen Oberfläche des Isolierkörpers nach innen versetzt und mit einer Hartlot-oder Sintermaterial- oder Weichlot-Schicht bedeckt. Diese Bedeckung ist derart ausgeführt, dass die bedeckende Materialschicht eine mit der gehäuseseitigen Oberfläche des Isolierkörpers plane oder aus der gehäuseseitigen Oberfläche des Isolierkörpers herausgewölbte Oberfläche hat. Bei praktisch realisierten Ausführungen hat eine patella-oder meniskusförmige bedeckende Materialschicht, je nach Größe der Durchführung, eine maximale Höhe gegenüber der gehäuseseitigen Oberfläche des Isolierkörpers im Bereich zwischen 0,01 und 1 mm.

In einer weiteren Ausführung der Erfindung ist das sekundäre Anschlusselement elektrisch leitend mit dem primären Anschlusselement gefügt und das sekundäre Anschlusselement besitzt mindestens eine weichlötbare Kontaktstelle. Hierbei ist das oder jedes sekundäre Anschlusselement mit dem entsprechenden primären Anschlusselement über ein Weichlot, niedrig schmelzendes Hartlot oder Sintermaterial verbunden, oder es besteht aus einem Weichlot, niedrig schmelzenden Hartlot oder Sintermaterial.

In praktisch relevanten Ausführungen ist das oder mindestens ein sekundäres Anschlusselement sphärisch, elliptisch oder polyedrisch geformt. In ein und derselben Durchführung können auch verschiedene geometrische Konfigurationen einzelner sekundärer Kontaktelemente unterschiedlich sein, um etwa speziellen Leiterkonfigurationen auf dem an die Durchführung anzubindenden Leitungsträger optimal Rechnung zu tragen.

Im Interesse erleichterter Prozessführung bei den Folgeschritten ist in weiteren Ausführungen mindestens eine aus der gehäuseseitigen Oberfläche des Isolierkörpers herausstehenden Oberfläche des oder jedes sekundären Anschlusselements mit einem die Weichlötbarkeit oder Sinterfähigkeit verbesserndem Material, insbesondere Cu, Ni, Ag, Sn. Au, Pt, Ir, Pd oder Legierungen aus mindestens zweien dieser oder einem die Weichlötbarkeit verbessernden Schichtsystem, wie etwa EPIG, ENEPIG, HAL oder dergleichen, beschichtet. Es versteht sich, dass das konkrete Beschichtungssystem entsprechend den Spezifika des Folgeprozesses zur Anbindung der Durchführung ausgewählt und optimiert wird.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische Darstellung eines herkömmlichen Herzschrittmachers,
- Fig. 2: eine Längsschnittdarstellung eines Ausführungsbeispiels der erfindungsgemäßen Durchführung,
- Fig. 3: eine Längsschnittdarstellung einer weiteren Ausführung der erfindungsgemäßen Durchführung, und
- Fig. 4: eine Längsschnittdarstellung einer weiteren Ausführung der erfindungsgemäßen Durchführung, und
- Fig. 5: eine schematische Querschnittsdarstellung einer weiteren erfindungsgemäßen Durchführung.

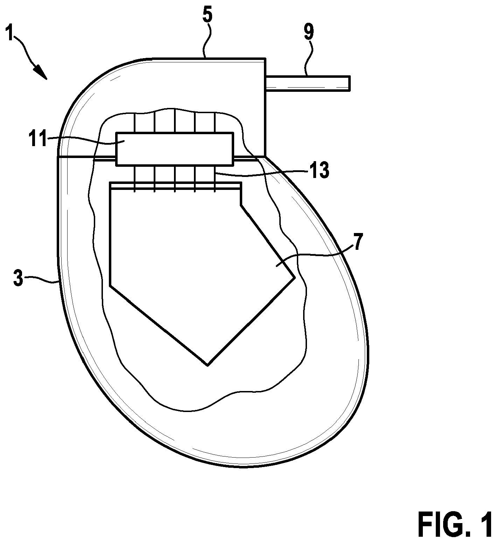

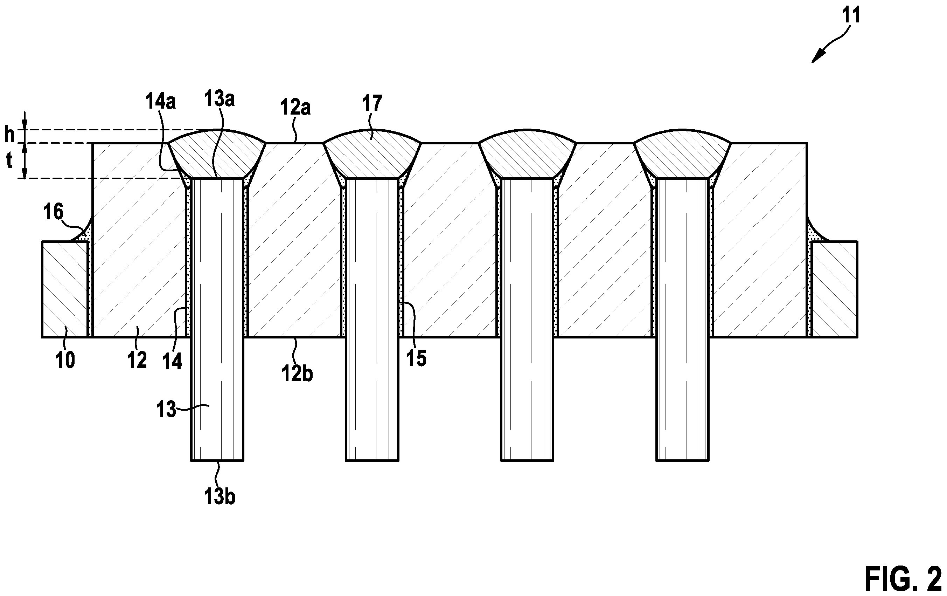

In allen Figuren 2 bis 5 ist die Durchführung, in Anlehnung an Fig. 1 und unabhängig von den abweichenden geometrischen Formen und Anordnungen ihrer Teile und Details, einheitlich mit Ziffer 11 bezeichnet, und die Bezeichnung einzelner Abschnitte oder der jeweiligen Beschichtung ist entsprechend gewählt.

Fig. 2 zeigt eine Durchführung 11 mit einem Isolierkörper 12 aus Isolationskeramik, der von einem Durchführungsflansch 10 umgeben ist und in den mehrere Anschlussstifte 13 eingesetzt sind. Die Verbindungen zwischen den Anschlussstiften 13 und den Wandungen der diese aufnehmenden Durchlässe bzw. Bohrungen 14 im Isolierkörper sowie zwischen dem Außenumfang des Isolierkörpers 12 und dem Innenumfang des Durchführungsflansches 10 sind jeweils durch ein Hartlotelement 15 bzw. 16 gebildet.

Die in ihrer Grundform zylindrischen Bohrungen 14 im Isolierkörper 12 sind zu einer ersten (im Gebrauchszustand gehäuseseitigen) Oberfläche 12a des Isolierkörpers mit konischen Erweiterungsabschnitten 14a versehen. Die Anschlussstifte 13 sind gegenüber der gehäuseseitigen Oberfläche 12a des Isolierkörpers 12 versenkt, wogegen die gegenüberliegenden Enden 13b der Anschlussstifte aus der gegenüberliegenden (headerseitigen) Oberfläche 12b des Isolierkörpers hervorstehen.

Über den zurückversetzten gehäuseseitigen Enden 13a der Anschlussstifte 13 ist ein Pfropfen 17 aus weich lötbarem Löt- oder Sintermaterial aufgebracht, der jeweils den konischen Erweiterungsabschnitt 14a der zugehörigen Bohrung über dem Hartlot 15 ausfüllt. Der Pfropfen 17 reicht bis zu einer Tiefe t von der gehäuseseitigen Oberfläche 12a in die Bohrung 14 hinein und wölbt sich aus der Oberfläche 12a kuppelförmig mit einer maximalen Höhe h auf. Er dient als Kontaktbereich für ein späteres Verlöten der Durchführung mit einer auf deren Gehäuseseite angeordneten Leiterplatte oder ähnlichem Element der im Gerätegehäuse eines IMD untergebrachten Elektronik.

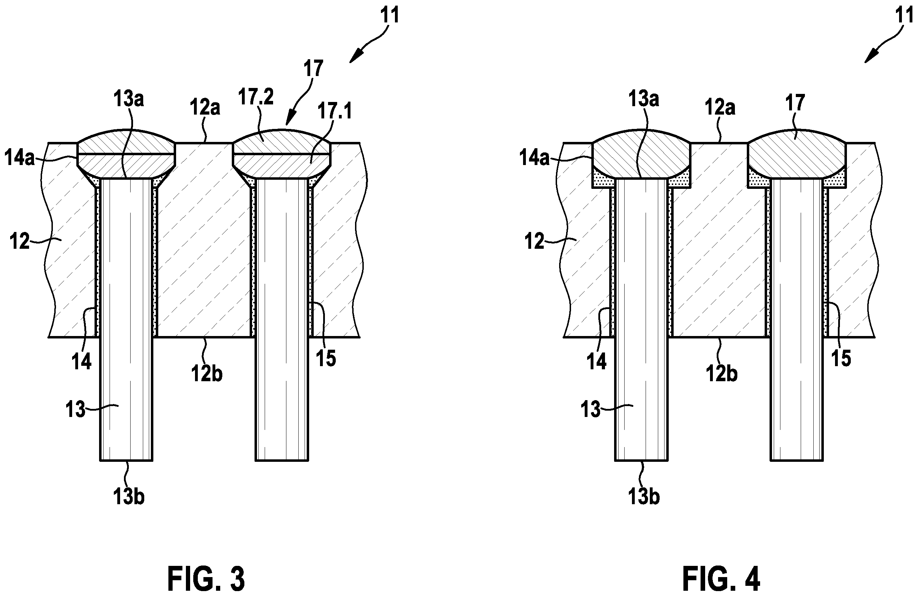

Fig. 3 zeigt schematisch eine weitere, kleinere Durchführung 11, kann aber auch als Ausschnittdarstellung einer größeren Durchführung verstanden werden, die mehr als die zwei in Fig. 3 gezeigten Anschlussstifte 13 umfasst. Ein Durchführungsflansch ist hier nicht gezeigt, kann aber vorhanden sein. Die Konfiguration der die Anschlussstifte aufnehmenden Bohrung 14 und die Positionierung der Anschlussstifte 13 ist ähnlich wie bei der Durchführung nach Fig. 2, mit folgenden Abweichungen:
Die Form der Erweiterungsabschnitte 14a an der gehäuseseitigen Oberfläche 12a des Isolierkörpers 12 ist hier nicht konisch, sondern die Erweiterungsabschnitte umfassen einen unteren konischen und einen oberen zylindrischen Abschnitt. Weiterhin ist in diesen Erweiterungsabschnitten und auf der gehäuseseitigen Stirnfläche 13a der Anschlussstifte 13 ein zweiteiliger Materialpfropfen 17 vorgesehen, der eine untere, auf der Anschlussstift-Stirnfläche 13a liegende Schicht 17.1 aus einem nicht weich lötbaren Lot- oder Sintermaterial und eine hierauf angebrachte, die untere Schicht abdeckende Schicht 17.2 aus einem weich lötbaren Lot- oder Sintermaterial umfasst.

Mit dem in den Erweiterungsabschnitt in der Schicht 17.1 eingebrachten (zweiten) Hartlot oder Sintermaterial, das einen niedrigeren Schmelzpunkt als das Hartlot der Hart-Verlötungen 15 hat, können Füllstandsunterschiede in den Bohrungen 14 ausgeglichen werden, die bei einzelnen Prozessschritten der Herstellung der Durchführung prozessbedingt auftreten können, womit wiederum unerwünschte Abweichungen der elektrischen Eigenschaften der Durchführung in den einzelnen Anschlussbereichen vermieden werden können. Das Material wird mittels Hartlöten oder Sintern an die Hartverlötungen 15 im Ringspalt zwischen Innenwandung der Bohrung 14 und Außenwandung des Anschlussstifts 13 angebunden.

Fig. 4 zeigt in einer weiteren Abwandlung eine Durchführung 11 mit wiederum ähnlichem Aufbau wie bei den Durchführungen nach Fig. 2 und 3. Jedoch sind hier die Erweiterungsabschnitte 14a der die Anschlussstifte 13 aufnehmenden Öffnungen bzw. Bohrungen 14 als stufenartige zylindrische Erweiterung ausgeführt, und die Erweiterung ist teilweise mit verbliebenem Material der Hart-Verlötungen 15 und zu einem anderen Teil (im oberen Bereich) mit einem Weichlot-Pfropfen 17 ausgefüllt.

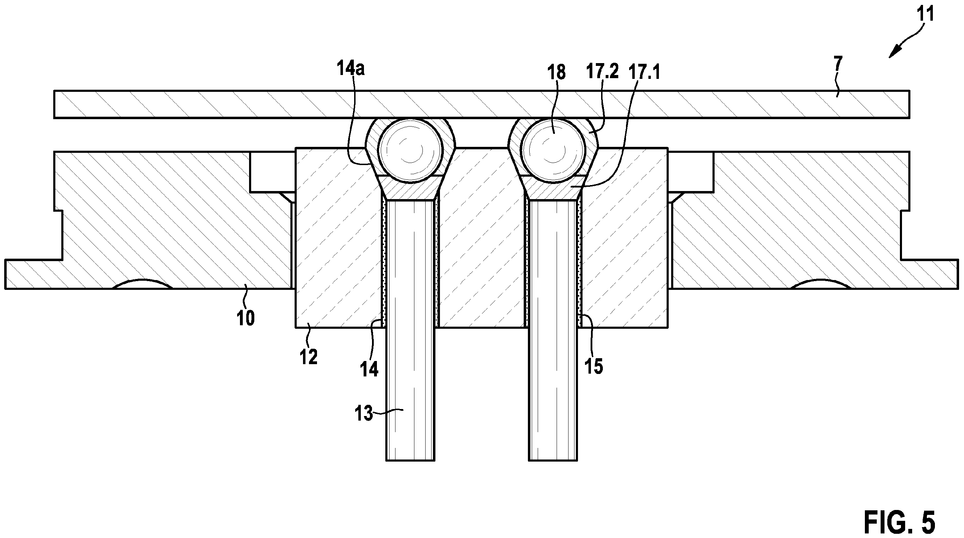

Fig. 5 zeigt in einer schematischen Querschnittsdarstellung eine weitere Durchführung 11, in Zuordnung zu einer Leiterplatte 7, an der sie montiert ist. Der grundsätzliche Aufbau der Durchführung 11 entspricht wieder den Ausführungen nach Fig. 2 bis 4, einschließlich des Vorsehens von Erweiterungsabschnitten 14a der Bohrungen 14 für die Anschlussstifte 13.

Jedoch ist hier in die Erweiterungsabschnitte 14a nicht nur ein erstes, nicht weich lötbares Lot- oder Sintermaterial 17.1 und ein zweites, weich lötbares Lot- oder Sintermaterial 17.2 eingebracht, sondern zusätzlich eine Metallkugel 18 als sekundäres Anschlusselement, neben dem Anschlussstift 13 vorgesehen, der in diesem Kontext als primäres Anschlusselement zu bezeichnen ist. Vermittels dieses Aufbaus ist die Durchführung besonders einfach mit der Technik des Reflow-Lötens mit korrespondierenden Leiterzügen auf der Leiterplatte 7 verbindbar.

Nach dem Hartlöten werden die Bohrungen 14 mit der bei niedriger Energie schmelzenden Hartlot-, Weichlot- oder einer Sinterpaste 17.1 aufgefüllt. Durch das Einfüllen einer pastösen Ausgleichsmasse in die Bohrungen, werden Niveauunterschiede aufgrund unterschiedlichen Aufschmelzverhaltens des Hartlotes in der Keramik ausgeglichen.

Auf die pastöse, niedrigschmelzende Hartlot-, Weichlot- oder Sinterpaste 17.1 wird das polyedrische, ovale oder runde sekundäre Anschlusselement 18 gesetzt. Diese Elemente 18 bestehen z. B. aus gut verfügbaren Materialen (z. B. Wolframcarbid, 1.4125). Die Oberfläche der Elemente 18 wird mittels galvanischem Verfahren veredelt und damit eine sehr gute Weichlot- bzw. Sinterfähigkeit hergestellt. Dies kann durch Beschichten mit Ni und Ag oder Ni und Sn erreicht werden. Ferner können aus der Literatur bekannte Oberflächenschichtsysteme (z. B. EPIG, ENEPIG, HAL) zum Einsatz kommen, die eine dauerhafte Lötbarkeit erreichen.

Die automatisierte Bestückung der sekundären Anschlusselemente ist sehr einfach möglich, da sie sich aufgrund ihrer Geometrie sehr einfach vereinzeln und zuführen lassen. Während der Bestückung verlorene Elemente rollen automatisch ab und müssen so nicht manuell oder mittels optischer Inspektion vom Produkt entfernt werden.

Durch das Fügen der Anschlusselemente wird eine weichlötbare und oberflächenmontierbare elektrische Durchführung für medizinische Zwecke geschaffen.

Aufgrund der einfachen Montage ist sowohl die manuelle als auch die automatisierte Herstellung mit großer Wirtschaftlichkeit möglich.

## Patentansprüche

1. Elektrische Durchführung (11) eines implantierbaren medizinelektronischen Geräts (1), das ein Gehäuse (3) und insbesondere ein Kopfteil (5) aufweist, mit einem Isolierkörper (12), der eine gehäuseseitige (12a) und eine dieser gegenüberliegende gehäuseabgewandte, insbesondere kopfteilseitige (12b), Oberfläche hat, einem den Isolierkörper umfassenden Durchführungsflansch (10) und mindestens einem den Isolierkörper durchstoßenden primären Anschlusselement (13), insbesondere mehreren primären Anschlusselementen, zum Anschluss eines im Gehäuse angeordneten elektrischen oder elektronischen Bauelements (7) des Gerätes, wobei das oder jedes primäre Anschlusselement mittels einer Hartlotverbindung (15) in einem Durchlass (14) des Isolierkörpers fluiddicht befestigt ist, wobei das oder jedes primäre Anschlusselement ein gehäuseseitiges Ende (13a) aufweist,
**dadurch gekennzeichnet, dass**
das gehäuseseitige Ende (13a) des oder jedes primären Anschlusselementes (13) gegenüber der gehäuseseitigen Oberfläche (12a) des Isolierkörpers (12) nach innen versetzt und mit einer Hartlot- oder Sintermaterial- (17.1) oder Weichlot-Schicht (17.2) derart bedeckt ist, dass die bedeckende Materialschicht eine mit der gehäuseseitigen Oberfläche des Isolierkörpers plane Oberfläche hat oder eine aus der gehäuseseitigen Oberfläche des Isolierkörpers herausgewölbte Kuppel bildet.

2. Durchführung nach Anspruch 1, wobei die Kuppel der bedeckenden Materialschicht (17.1, 17.2) eine maximale Höhe gegenüber der gehäuseseitigen Oberfläche (12a) des Isolierkörpers (12) im Bereich zwischen 0,01 und 1,0 mm hat.

3. Durchführung nach einem der vorangehenden Ansprüche, wobei das sekundäre Anschlusselement (18) elektrisch leitend mit dem primären Anschlusselement (13) gefügt ist und das sekundäre Anschlusselement mindestens eine weichlötbare Kontaktstelle besitzt.

4. Durchführung nach Anspruch 3, wobei das oder jedes sekundäre Anschlusselement (18) mit dem entsprechenden primären Anschlusselement (13) über ein Weichlot, niedrig schmelzendes Hartlot oder Sintermaterial (17.1, 17.2) verbunden ist oder aus einem Weichlot, niedrig schmelzendes Hartlot oder Sintermaterial besteht.

5. Durchführung nach Anspruch 3 oder 4, wobei das oder mindestens ein sekundäres Anschlusselement (18) sphärisch, elliptisch oder polyedrisch geformt ist.

6. Durchführung nach einem der Ansprüche 3 bis 5, wobei mindestens eine aus der gehäuseseitigen Oberfläche des Isolierkörpers herausstehende Oberfläche des oder jedes sekundären Anschlusselements (18) mit einem die Weichlötbarkeit oder Sinterfähigkeit verbesserndem Material, insbesondere Cu, Ni, Ag, Sn, Au, Pt, Ir, Pd oder Legierungen aus mindestens zweien dieser oder einem die Weichlötbarkeit verbessernden Schichtsystem, wie EPIG, ENEPIG oder HAL, beschichtet ist.

7. Implantierbares medizinelektronisches Gerät mit einer Durchführung nach einem der vorangehenden Ansprüche, insbesondere ausgeführt als Herzschrittmacher, implantierbarer Kardioverter oder Cochlear-Implantat.

## Claims

1. An electrical feedthrough (11) of an implantable medical electronic device (1) having has a housing (3) and, in particular, a header (5), comprising an insulating body (12) including a housing-side surface (12a) and an surface, in particular, a header-side surface (12b), located opposite the housing-side surface and facing away from the housing, a feedthrough flange (10) surrounding the insulating body, and at least one primary connection element (13), and in particular a plurality of primary connection elements, penetrating the insulating body for connecting an electrical or electronic component (7) of the device disposed in the housing, the or each primary connection element being attached, by means of a hard solder connection (15), in a passage (14) of the insulating body in a fluid-tight manner, the or each primary connection element having a housing-side end (13a),
**characterized in that**
the housing-side end (13a) of the or each primary connection element (13) is inwardly offset in relation to the housing-side surface (12a) of the insulating body (12) and covered with a hard solder or sintered material layer (17.1) or soft solder layer (17.2) so that the covering material layer has a surface that is even with the housing-side surface of the insulating body or forms a dome bulging up out of the housing-side surface of the insulating body.

2. The feedthrough according to claim 1, wherein the dome of the covering material layer (17.1, 17.2) has a maximum height with respect to the housing-side surface (12a) of the insulating body (12) in a range between 0.01 and 1.0 mm.

3. The feedthrough according to any one of the preceding claims, wherein the secondary connection element (18) is electrically conductively joined to the primary connection element (13), and the secondary connection element has at least one soft solderable contact point.

4. The feedthrough according to claim 3, wherein the or each secondary connection element (18) is connected to the corresponding primary connection element (13) by a soft solder, a hard solder having a low melting temperature, or a sintered material (17.1, 17.2), or is made of a soft solder, hard solder having a low melting temperature or sintered material.

5. The feedthrough according to claim 3 or 4, wherein the or at least one secondary connection element (18) is spherically, elliptically or polyhedrally shaped.

6. The feedthrough according to any one of claims 3 to 5, wherein at least one surface of the or each secondary connection element (18) protruding from the housing-side surface of the insulating body is coated with a material improving soft-solderability or sinterability, in particular Cu, Ni, Ag, Sn, Au, Pt, Ir, Pd, or alloys of at least two of these, or a layer system improving soft-solderability, such as EPIG, ENEPIG or HAL.

7. An implantable medical electronic device comprising a feedthrough according to any one of the preceding claims, in particular designed as a cardiac pacemaker, implantable cardioverter or cochlear implant.

## Revendications

1. Conduite électrique (11) d'un appareil médical électronique implantable (1) qui présente un boîtier (3) et en particulier une partie de tête (5), avec un corps isolant (12) qui a une surface du côté du boîtier (12a) et une surface opposée au boîtier située en face de celle-ci, notamment une surface du côté partie de tête (12b), une bride de conduite (10) comprenant le corps isolant et au moins un élément de connexion primaire (13) traversant le corps isolant, notamment plusieurs éléments de connexion primaires pour la connexion à un composant électrique ou électronique (7) de l'appareil disposé dans le boîtier, où l'élément primaire ou chaque élément de connexion primaire est fixé de manière étanche aux fluides dans un passage (14) du corps isolant au moyen d'une liaison par brasage fort (15), où l'élément ou chaque élément de connexion primaire présente une extrémité du côté boitier (13a),
**caractérisée en ce que**
l'extrémité du côté boîtier (13) de l'élément ou de chaque élément de connexion primaire (13) est décalée vers l'intérieur par rapport à la surface du côté boîtier (12a) du corps isolant (12) et est recouverte avec une couche de matière de brasure fort ou de frittage (17.1) ou avec une couche de brasage tendre (17.2) de telle manière que la couche de matière de recouvrement a une surface plane avec la surface du côté du boîtier du corps isolant ou forme une coupole s'élevant à partir de la surface du côté boîtier du corps isolant.

2. Conduite selon la revendication 1, dans laquelle la coupole de la couche de matière (17.1, 17.2) de recouvrement a une hauteur maximale par rapport à la surface du côté boîtier (12a) du corps isolant (12) dans la plage entre 0,01 et 1 mm.

3. Conduite selon l'une des revendications précédentes, dans laquelle l'élément de connexion secondaire (18) électriquement conducteur est ajouté à l'élément de connexion primaire (13) et l'élément de connexion secondaire possède au moins un point de contact pouvant être brasé de manière tendre.

4. Conduite selon la revendication 3, dans laquelle l'élément secondaire ou chaque élément de connexion secondaire (18) est relié avec l'élément de connexion primaire (13) correspondant par le biais d'un brasage tendre, d'une matière de brasage fort à point de fusion faible ou une matière de frittage (17.1, 17.2) ou est constitué d'une matière de brasage tendre, d'une matière de brasage fort à faible point de fusion ou d'une matière de frittage.

5. Conduite selon la revendication 3 ou la revendication 4, dans laquelle l'élément secondaire ou au moins un élément de connexion secondaire (18) est de forme sphérique, elliptique ou polyédrique.

6. Conduite selon l'une des revendications 3 à 5, dans laquelle au moins une surface dépassant de la surface du côté boîtier du corps isolant de l'élément secondaire ou de chaque élément de connexion secondaire (18) est revêtue avec un matériau améliorant le brasage tendre ou le frittage, notamment avec du Cu, ni, Ag, Sn, Au, Pt, Ir, Pd ou des alliages à base d'au moins deux métaux parmi ceux-ci ou avec un système de couche améliorant le brasage tendre comme des systèmes à l'EPIG, à l'ENEPIG ou à l'HAL.

7. Appareil médical électronique implantable doté d'une conduite selon l'une des revendications précédentes, notamment conçu sous forme de stimulateur cardiaque, de cardioverteur implantable ou d'implant cochléaire.
